# EUROPEAN PATENT SPECIFICATION

(11) **EP 2 083 812 B1**
(45) Date of publication and mention of the grant of the patent: **05.04.2017**
(21) Application number: 07819483.4
(22) Date of filing: 30.10.2007
(51) Int. Cl.: A61K 31/198, A61K 31/69, A61P 11/06, A61P 37/08

(54) **USE OF ARGINASE INHIBITORS IN THE TREATMENT OF ASTHMA AND ALLERGIC RHINITIS**
VERWENDUNG VON ARGINASE-HEMMERN BEI DER BEHANDLUNG VON ASTHMA UND ALLERGISCHER RHINITIS
UTILISATION D'INHIBITEURS DE L'ARGINASE DANS LE TRAITEMENT DE L'ASTHME ET DE LA RHINITE ALLERGIQUE

(30) Priority: 21.11.2006 EP 06024111
(43) Date of publication of application: 05.08.2009
(73) Proprietor: Rijksuniversiteit Groningen, 9712 CP Groningen (NL)
(72) Inventor: MEURS, Hermanus, 9892 PH Feerwerd (NL); ZAAGSMA, Johan, 9722 RS Groningen (NL); MAARSINGH, Harm, 9741 KK Groningen (NL); VAN DUIN, Michel, 5340 BH Oss (NL)
(74) Representative: van Someren, Petronella F. H. M.
(86) International application number: PCT/EP2007/009449
(87) International publication number: WO 2008/061612

(56) References cited:
- WO-A-99/58095
- WO-A-03/073990
- WO-A-2004/073623
- WO-A-2005/003164
- RICCIARDOLO F L M ET AL: "The therapeutic potential of drugs targeting the arginase pathway in asthma" EXPERT OPINION ON INVESTIGATIONAL DRUGS 2005 UNITED KINGDOM, vol. 14, no. 10, 2005, pages 1221-1231, XP002463287 ISSN: 1354-3784
- H. Maarsingh ET AL: "Increased arginase activity contributes to airway remodelling in chronic allergic asthma", EUROPEAN RESPIRATORY JOURNAL., vol. 38, no. 2, 10 February 2011 (2011-02-10), pages 318-328, XP055301092, DK ISSN: 0903-1936, DOI: 10.1183/09031936.00057710

## Description

The present invention relates to the prophylactic maintenance treatment of allergic asthma, non-allergic asthma and allergic rhinitis.

Allergic asthma is a chronic inflammatory disease of the airways. Characteristic features of this disease are allergen-induced early and late bronchial obstructive reactions, associated with infiltration and activation of inflammatory cells in the airways - particularly Th2 lymphocytes and eosinophils - and the development of airway hyperresponsiveness to a variety of stimuli, including allergens, chemical irritants, cold air and pharmacological agents like histamine and methacholine.

Alterations in the neurogenic and nonneurogenic control of airway smooth muscle function, as well as physical changes in the airways, including epithelial damage, thickening of the mucosa due to edema and local vasodilation, and mucous secretions in the airway lumen, may be involved in the development of airway hyperresponsiveness after the early and late asthmatic reactions in acute asthma.

In chronic asthma, airway remodelling due to irreversible structural changes of the airway wall, including thickening of the basement membrane, mucous gland hypertrophy, subepithelial fibrosis and increased airway smooth muscle mass, may be importantly involved in persistent airway hyperresponsiveness and decline of lung function.

All of these changes are induced by a complex cascade of inflammatory reactions involving various allergic mediators, neurotransmitters, cytokines, growth factors and reactive oxygen and nitrogen species.

Allergic rhinitis is a collection of symptoms, predominantly in the nose, caused by airborne particles of dust, dander, or plant pollens in people who are allergic to these substances. When these symptoms are caused by pollen, the allergic rhinitis is commonly known as "hay fever". Allergic rhinitis is a common problem that may be associated with asthma. In patients with asthma, uncontrolled rhinitis seems to make asthma worse.

Due to the relatively high incidence of asthma in the population, the economic burden resulting from absence of work or even incapacity for work as well as for direct medical expenditures and medications is high. It is therefore desirable to prevent the symptoms. In addition, the quality of life of asthma and allergic patients would be highly improved in case the development of symptoms could be avoided by means of a prophylactic treatment.

It is therefore the object of the present invention to provide a novel prophylactic maintenance treatment for asthma and allergic rhinitis.

The research that led to the present invention for the first time demonstrated the importance of arginase in the pathophysiology of asthma *in vivo.* Using a guinea pig model of allergic asthma, the present inventors demonstrated that inhalation of the specific, isoenzyme-nonselective arginase inhibitor ABH acutely reversed the allergen-induced airway hyperresponsivenes (AHR) after the allergen-induced early (EAR) and late (LAR) asthmatic reaction, while pretreatment with the arginase inhibitor considerably reduced the sensitivity of the airways to inhaled allergen and protected against the development of allergen-induced EAR and LAR, and AHR after both reactions.

It was more specifically found that pretreatment with inhaled ABH caused a considerable reduction in sensitivity of the airways to the inhaled allergen (prophylaxis) . Pretreatment with ABH protected against the development of the allergen-induced bronchial obstructions and AHR when the animals were challenged with an allergen dose that provoked airway obstruction in saline-treated controls.

Furthermore, when animals were challenged to airway obstruction using a higher dose of the allergen than saline-treated controls, pretreatment with inhaled ABH still protected against the development of AHR after the early and late asthmatic reaction. In addition, ABH acutely reversed established allergen-induced AHR after the early as well as after the late asthmatic reaction.

The above effects of arginase inhibition may be explained by an increase of NO production in the airways, due to enhanced bioavailability of L-arginine to cNOS and/or iNOS, thereby attenuating allergen-induced mast cell activation, and inducing bronchoprotection against contractile stimuli. Particularly after the late asthmatic reaction the latter effect will also involve reduced iNOS-mediated production of peroxynitrite, the procontractile and proinflammatory metabolite of NO.

The above-mentioned mechanism of action is attractive as it combines anti-allergic (and anti-inflammatory) properties with (acute) bronchoprotection against contractile stimuli. Moreover, based on inhibition of arginase-mediated synthesis of polyamines and L-proline, ABH can inhibit airway remodeling associated with irreversible decline in lung function in chronic asthma.

ABH is particularly effective in patients with allergic asthma. Given the protective effect towards both allergen-induced bronchial obstruction and allergen-induced AHR, inhalation therapy with ABH is primarily directed to patients with persistent allergic asthma who regularly use (short acting) bronchodilators to treat symptoms, i.e. prophylactic maintenance treatment to prevent allergen-induced bronchial obstruction and development of AHR. In asthma therapy, AHR is a key feature to be targeted, as it is a major determinant of asthma severity. Therapy with ABH is intended for patients with mild, moderate as well as severe persistent asthma, as studies of Morris et al. (Am. J. Respir. Crit. Care Med. 2004 ; 170 : 148-153) have indicated an important role of arginase in patients with severe asthma exacerbations.

Given its anti-allergic effect, beneficial effects of ABH in patients with allergic rhinitis (often associated with allergic asthma) are also part of this invention.

Described herein is the use of an arginase inhibitor for the preparation of a medicament for the prophylactic maintenance therapy of an asthmatic or allergic patient by preventing obstruction of the upper and lower airways, in particular allergen-induced bronchial obstruction and/or airway hyperresponsiveness.

The invention relates to an arginase inhibitor for use in the prophylactic maintenance
therapy of an asthmatic or allergic patient by reducing the sensitivity of the airways to inhaled allergen and protecting against the development of allergen-induced early asthmatic reaction (EAR) and late asthmatic reaction (LAR), wherein the arginase inhibitor is administered by topical inhalation.

The patient to be treated is in particular an allergic patient having elevated arginase activity as a symptom or cause of his disorder. Patients having elevated arginase activity are usually asthmatic or have allergic rhinitis, but may also be non-allergic asthmatic patients having elevated arginase activity in the airways as a symptom or cause of this disorder.

In principle, any arginase inhibitor may be used according to the invention. Preferred arginase inhibitors for use in the invention are those described in US-6,723,710 having the formula:

HOOC-CH (NH₂) -X¹-X²-X³-X⁴-B (OH)₂

wherein each of X¹, X², X³' X⁴ is selected from the group consisting of -(CH₂)-, -S-, -0-, -(NH)-, and -(N-alkyl). The term "alkyl" as used herein refers to any C₁ to C₂₀ carbon backbone, linear or branched, unsubstituted or substituted, for example with F, S, O, or N, and pharmaceutically acceptable salts thereof.

In a preferred embodiment, the present invention relates to the use of arginase inhibitors according to the above general formula, wherein one of X¹, X², X³ or X⁴ is selected from the group consisting of -S-, -O-, -(NH)-, and the remainder of X¹, X², X³ or X⁴ are -(CH₂)-, and pharmaceutically acceptable salts thereof.

According to a particularly preferred embodiment, the present invention relates to the use of an arginase inhibitor according to the formula:

HOOC-CH(NH₂)-(CH₂)-(CH₂)-(CH₂)-(CH₂)-B(OH)₂

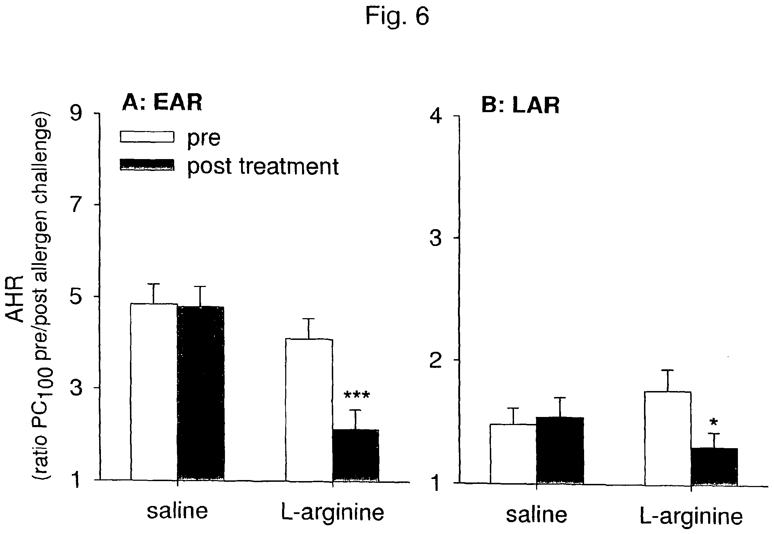

and pharmaceutically acceptable salts thereof. This compound is also designated as 2 (S) -amino-6-boronohexanoic acid or ABH. The preparation of ABH and corresponding compounds is for example described in US 6,723,710, especially as described in column 25, lines 34 to column 26, line 67, and Fig. 6.

Other preferred arginase inhibitors for use according to the present invention are those compounds selected from the group consisting of Nω-OH-L-arginine (NOHA), Nω-hydroxy-nor-L-arginine (nor-NOHA), α-difluoromethylornithine (DFMO), L-norvaline, iodoacetyl-L-ornithine, iodoacetyl-L-lysine, L-lysine, and L-citrulline and pharmaceutically acceptable salts thereof.

Preferably, the arginase inhibitor is used as the only active ingredient in the medicament. The arginase inhibitor, in particular ABH, can be used as such without being combined with other active ingredients. The arginase inhibitor is however combined with suitable non-active additives in a prophylactic formulation.

WO2004/073623 discloses the treatment of conditions associated with elevated arginine, including asthma, by means of arginine that is optionally combined with an arginine inhibitor and/or magnesium. This publication relates to treatment, not to prophylaxis. The basic treatment is with arginine. The arginase inhibitor may be added as a further active ingredient but is never the main or only active ingredient.

WO 2005/003164 discloses the use of sIL-13Ra2.Fc for inhibiting allergen-induced mucus production and airway hyperresponsiveness in Balb/C-treated and untreated mice sensitized with OVA. The asthmatic symptoms are however not completely prevented by this IL-13 antagonist. The prophylactic medicament of the invention is administered by inhalation. Inhalation formulations may for example provide the active ingredient in the form of an aerosol, droplets of a solution or suspension, or a powder and can be administered by means of an inhalation device. Additives may comprise surface active agents, preservatives, flavoring agents, buffering agents etc. The concentration of the active ingredient is for example selected from the range of 0.1 µg to 5 mg/inhalation, particularly 1 µg to 2 mg/inhalation, more particularly 10 µg to 1 mg/inhalation. A suitable dose for other administration routes comprises 0.0001 to 25 mg per kg body weight of the active ingredient.

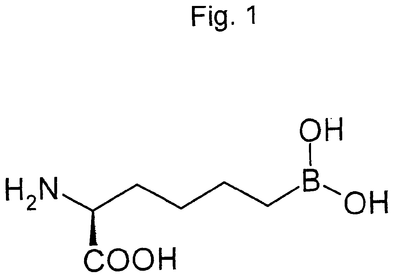

The CA index name of ABH is 6-borono-L-norleucine . Its structural formula is shown in Figure 1.

The present invention will be further illustrated in the Example that follows. In the Example reference is made to the following figures.
**Figure 1****:** Structural formula of ABH.

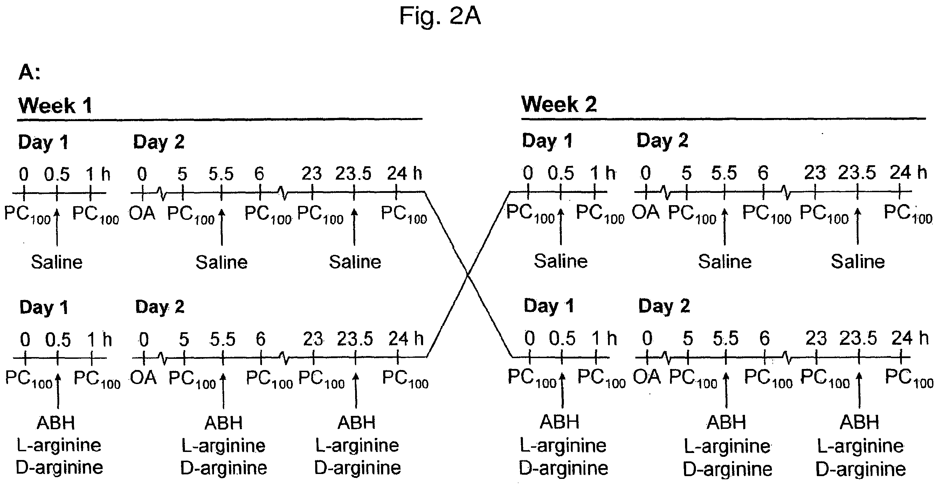

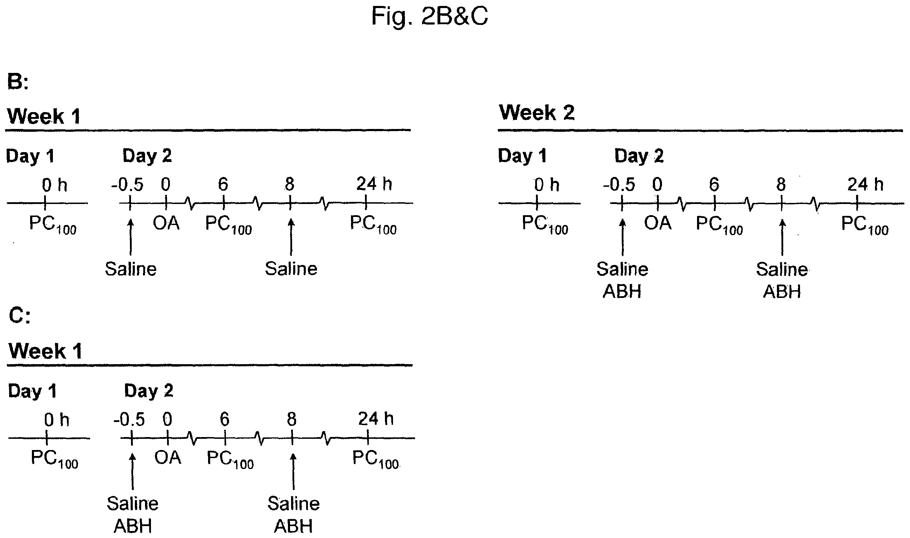

**Figure 2****:** Schematic illustration of the protocols used in this study. OA: ovalbumin challenge; PC₁₀₀: histamine PC₁₀₀ measurement.

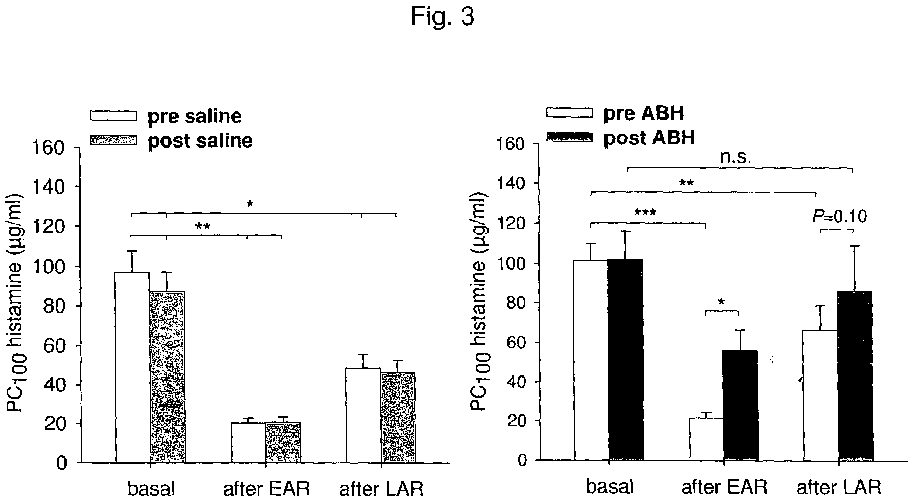

**Figure 3****:** Effects of inhalation of saline (left panel) or the arginase inhibitor ABH (25 mM nebulizer concentration; right panel) on basal airway responsiveness toward inhaled histamine and on histamine hyperresponsiveness after the EAR and LAR. Two subsequent PC₁₀₀-measurements were performed 30 min before (open bars) and 30 min after (filled bars) inhalation of saline or ABH. Data represent means ± SEM of 3-5 animals. *P<0.05, **P<0.01 and ***P<0.0001; n.s. = not significant.

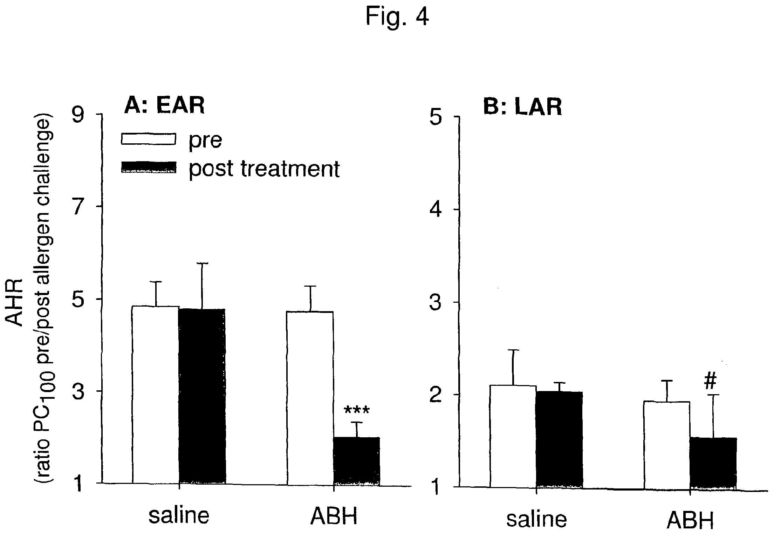

**Figure 4****:** Effects of inhalation of ABH (25 mM nebulizer concentration) on the AHR after the EAR (panel A) and the LAR (panel B) . AHR is defined as the ratio of the histamine PC₁₀₀ values obtained before (basal) and after allergen challenge (after EAR or LAR, respectively). Data represent means ± SEM of 3-5 animals. ***P<0.001 and *P=O.10 versus pretreatment.

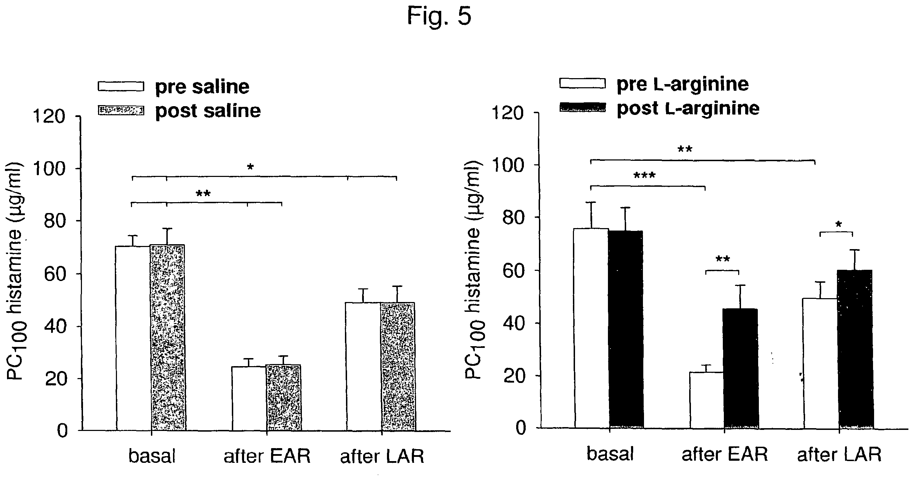

**Figure 5****.** Effects of inhalation of saline (left panel) or L-arginine (1 M nebulizer concentration; right panel) on basal airway responsiveness toward inhaled histamine and on histamine AHR after the EAR and the LAR. Two subsequent PC₁₀₀-measurements were performed 30 min before (open bars) and 30 min after (filled bars) inhalation of saline or L-arginine. Data represent means ± SEM of 9 animals. *P<0.05, **P<0.01 and ***P<0.0001.
**Figure 6****:** Effects of inhalation of L-arginine (1M nebulizer concentration) on the AHR after the EAR (panel A) and the LAR (panel B) . AHR is defined as the ratio of the histamine PC₁₀₀ values obtained before (basal) and after allergen challenge (after EAR or LAR, respectively). A value of 1 represents normoresponsiveness. Data represent means ± SEM of 9 animals. *P<0.05 and ***P<0.001 versus pretreatment.

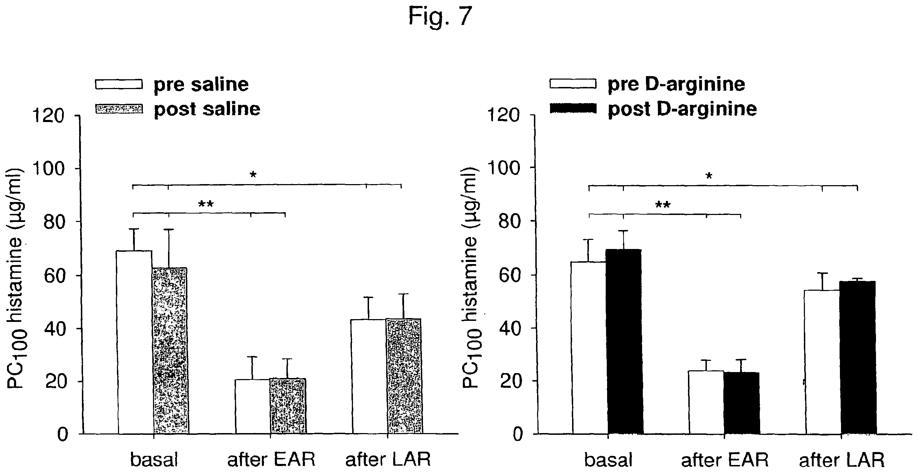

**Figure 7****:** Effects of inhalation of saline (left panel) or D-arginine (1 M nebulizer concentration; right panel) on basal airway responsiveness toward inhaled histamine and on histamine AHR after the EAR and the LAR. Two subsequent PC₁₀₀-measurements were performed 30 min before (open bars) and 30 min after (filled bars) inhalation of saline or D-arginine. Data represent means ± SEM of 3 animals. *P<0.05 and **P<0.01.

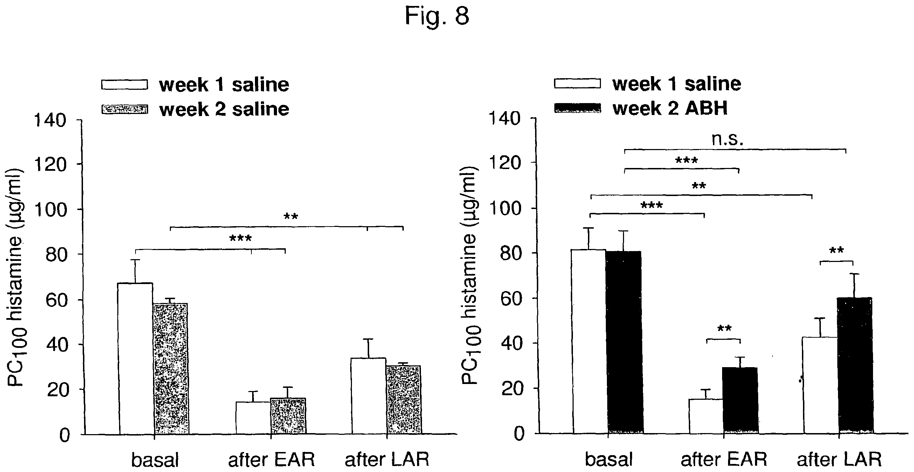

**Figure 8****:** Effects of inhalation of saline (filled bars, left panel) or ABH (25 mM; nebulizer concentration; filled bars, right panel) at 0.5 h before and 8 h after allergen inhalation on airway responsiveness to histamine after the EAR (6 h) and LAR (24 h) in comparison with saline effects obtained in the same animals one week earlier (open bars) . Data represent means ± SEM of 5 animals. *P<0.05 and ***P<0.001, n.s. = not significant.

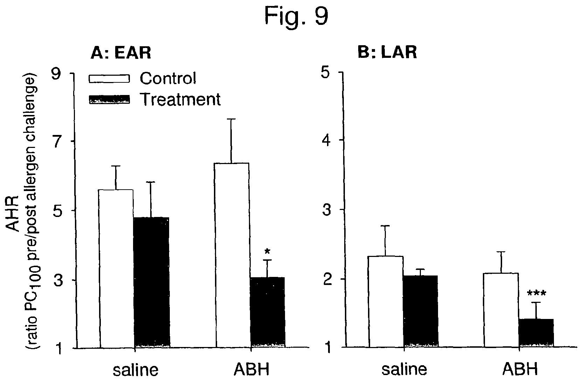

**Figure 9****:** Effects of inhalation of saline or ABH (25 mM nebulizer concentration) at 0.5 h before and 8 h after allergen inhalation on AHR to histamine after the EAR (filled bars, panel A) and the LAR (filled bars, panel B) in comparison with saline controls (open bars) obtained in the same animals one week earlier. AHR is defined as the ratio of the histamine PC₁₀₀ values obtained before (basal) and after allergen challenge (after EAR and LAR, respectively). A value of 1 represents normoresponsiveness. Data represent means ± SEM of 5 animals. *P<0.05 and ***P<0.001 versus control.

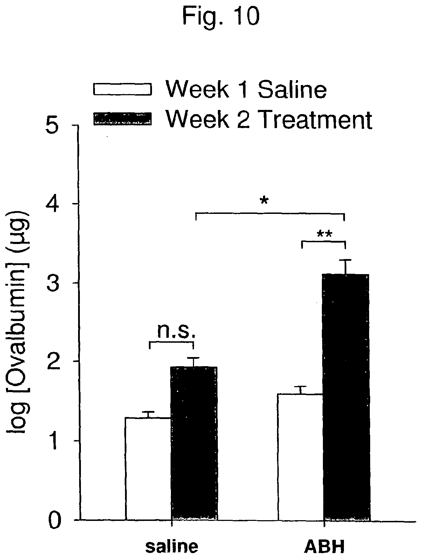

**Figure 10****:** Effects of pretreatment with saline or ABH (25 mM nebulizer concentration) on the ovalbumin dose required to induce airways obstruction (filled bars) compared to saline controls (open bars) obtained in the same animals one week earlier. Please note that ovalbumin dose is plotted logarithmically. Data represent means ± SEM of 5 animals. *P<0.05 and **P<0.01; n.s. = not significant.

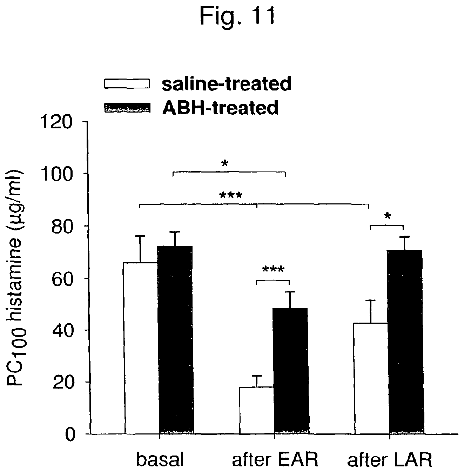

**Figure 11****:** Effects of treatment with inhaled saline or ABH (25 mM; nebulizer concentration) at 0.5 h before and 8 h after allergen inhalation on airway responsiveness to histamine after the EAR (6 h after allergen challenge) and LAR (24 h after allergen challenge). Both treatment groups were challenged with the same allergen dose, which induced airways obstruction in the saline- treated animals. Data represent means ± SEM of 5-6 animals. *P<0.05 and ***P<0.001.

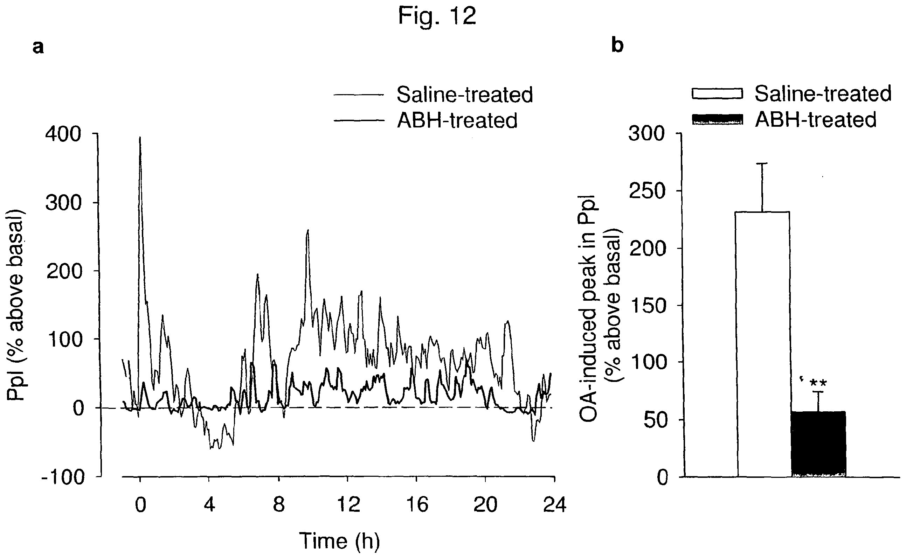

**Figure 12****:** (a) Representative on-line registrations of Pp1 in conscious, unrestrained guinea pigs after allergen challenge (t=0 h) . Animals were treated with either saline or 25 mM ABH (nebulizer concentration) 0.5 h before and 8 h after allergen exposure. Both treatment groups were exposed to the same allergen dose, (b) Effects of pretreatment with saline or ABH on the initial peak rise after allergen challenge. Data represent means ± SEM of 5-6 animals. **P<0.01 compared to saline-treated animals.

### EXAMPLE

### INTRODUCTION

Using a guinea pig model of allergic asthma, this example reports *in vivo* data demonstrating that inhalation of ABH acutely reverses allergen-induced AHR after the EAR and LAR, which can be mimicked by L-arginine. Moreover, it is demonstrated that pretreatment with ABH considerably reduces the sensitivity of the airways to inhaled allergen and protects against the development of allergen-induced EAR and LAR, and AHR after both reactions.

### METHODS

### Animals and sensitization procedure

Outbred male specified pathogen-free Dunkin Hartley guinea pigs (Harlan Heathfield, UK) were used in this example. All animals, weighing approximately 250 g, were actively IgE-sensitized to ovalbumin as described by Van Amsterdam et al. [Agents Actions 1989, 26:48-51]. The animals were operated on 2 weeks after sensitization and used experimentally in weeks 4 and 5 after sensitization. The animals were housed in individual cages in climate-controlled animal quarters and given water and food ad libitum, while a 12-h on/12-h off light cycle was maintained. All protocols described in this study were approved by the University of Groningen Committee for Animal Experimentation.

### Measurement of airway function

Airway function was assessed by on-line measurement of pleural pressure (Pₚₗ) under unrestrained conditions as described previously [Santing et al., PuIm Pharmacol 1992, 5:265-272; Meurs et al. , Nature Protocols 2006, 1:840-847].

In short, a small fluid-filled latex balloon catheter was surgically implanted inside the thoracic cavity. The free end of the catheter was driven subcutaneously to the neck of the animal, where it was exposed and attached permanently. Via an external fluid-filled cannula, the pleural balloon catheter was connected to a pressure transducer (TXX-R, Viggo-Spectramed, Bilthoven, Netherlands). Pₚₗ (in cm H₂O) was measured continuously using an on-line computer system.

Using a combination of flow measurement with a pneumotachograph, implanted in the trachea, and pressure measurement with the pleural balloon catheter, it was shown that changes in Pₚₗ are linearly related to changes in airway resistance and hence can be used as a sensitive index for allergen- and histamine-induced bronchoconstriction [Santing et al., supra], In this way, airway function can be monitored repeatedly and continuously for prolonged periods of time, while the animals are unaware of the measurements being taken.

During the experimental protocol (1-4 weeks after surgery) baseline Pₚₗ-measurements remained stable and no signs of inflammation were observed at the sites of surgery.

### Provocation procedures

Allergen and histamine provocations were performed by inhalation of aerosolized solutions. These provocations were carried out in a specially designed perspex cage of 9 1, in which the guinea pigs could move freely as previously described [Santing et al.; Meurs et al. , both supra], A DeVilbiss nebulizer (type 646) driven by an airflow of 8 1/min provided the aerosol with an output of 0.33 ml/min. The animals were habituated to the experimental conditions and the provocations procedure at least one week after surgery, when preoperative weight was restored, as described previously [Meurs et al . , supra].

On the experimental days following the habituation procedure, allergen and/or histamine provocations were performed as described below. All provocations were preceded by an adaptation period of at least 30 min, followed by two consecutive control provocations with saline, each provocation lasting 3 min and separated by 7 min intervals. A baseline Pₚₗ-value was calculated by averaging the Pₚₗ-values from the last 20 min of the adaptation period.

In order to assess the airway reactivity for histamine, provocations with increasing concentration steps (6.25, 12.5, 25, 50, 75, 100 and 125 µg/ml) in saline were performed. Histamine provocations lasted 3 min and were separated by 7 min intervals. Animals were challenged until Pₚₗ was increased by more than 100% above baseline for at least 3 consecutive minutes. The concentration of histamine causing a 100% increase of Pₚₗ (PC₁₀₀) was derived by linear interpolation of the concentration-Pₚₗ curve and was used as an index for airway reactivity towards histamine. Pₚₗ returned to baseline within 15 min after the last histamine provocation.

Allergen provocations were performed by inhalation of increasing concentrations of 0.5, 1.0 or 3.0 mg/ml ovalbumin in saline and were discontinued when the first signs of respiratory distress were observed and an increase in Pₚₗ of more than 100% was reached.

### EXPERIMENTAL PROTOCOLS

### Reversal of allergen-induced AHR by ABH and L-arginine

On two different occasions, separated by one week interval, guinea pigs were treated either with vehicle (saline) or drug (ABH, L-arginine or D-arginine), to establish the acute effects of these drugs on basal airway responsiveness to histamine as well as on allergen-induced airway hyperresponsiveness (AHR) after the EAR and the LAR (Fig. 2).

On the first experimental day, saline- or drug-induced effects on basal histamine reactivity were established. Thirty minutes after the assessment of the basal histamine PC₁₀₀, an aerosol of saline (control) or 25 mM ABH, 1.0 M L-arginine or 1.0 M D-arginine (nebulizer concentrations) was inhaled for 15 min. Following these inhalations, a second histamine PC₁₀₀-measurement was performed starting 15 min later. On the second day allergen provocations were performed. At 5 h and 23 h after ovalbumin provocation, histamine PC₁₀₀ values were measured to determine the allergen-induced AHR after the EAR and the LAR, respectively.

Saline, ABH and L- or D-arginine inhalations were performed at 5.5 h and 23.5 h after ovalbumin provocation, and subsequent histamine PC₁₀₀-values were reassessed at 6 h and 24 h after the allergen provocation. Saline- and drug inhalations were alternated with one week interval in a random cross-over design (Fig. 2).

### Prevention of allergen-induced AHR by ABH

On the first experimental day, the basal histamine PC₁₀₀ was assessed. The next day, saline was inhaled for 15 min, 0.5 h prior to and at 8 h after allergen inhalation, and histamine PC₁₀₀ measurements were performed 6 and 24 h post allergen challenge. One week later, the same protocol was repeated with inhalations of either 25 mM ABH (nebulizer concentration) or saline at t = -0.5 and 8 h. All animals were challenged with increasing doses of allergen (0.5, 1 and 3 mg/ml) until obstruction.

In a second experiment, animals were challenged once with allergen. On the first experimental day, the basal histamine PC₁₀₀ was assessed. The next day, animals were pretreated with either saline or ABH (25 mM; nebulizer concentration) 0.5 h before and 8 h after allergen inhalation. The ABH-pretreated animals were challenged with the same allergen dose that caused airway obstruction in the saline-treated guinea pigs. In this protocol, allergen-induced EAR (between 0 and 5 h after allergen provocation) and LAR (between 8 h and 24 h after allergen provocation) were measured by continuous Pₚₗ registration, and airway reactivity to histamine was assessed at 6 h and 24 h after allergen challenge (Fig. 2).

### Data analysis

The sensitivity to inhaled allergen was expressed as the total amount (mg) of allergen nebulized to obtain airway obstruction, which is the factor of the nebulized time (s), the allergen dose in the nebulizer (mg/ml) and the aerosol output (ml/min).

The magnitudes of the EAR and the LAR were expressed as the area under the Pₚₗ time-response curve (AUC) between 0 and 5 h (EAR) and between 8 and 24 h (LAR) after allergen provocation. Pₚₗ was expressed as percent change from baseline and AUC was calculated by trapezoid integration over discrete 5 min time periods (Santing et al. J. Allergy Clin. Immunol. 1994, 93:1021-1030).

All data are expressed as means ± SEM. Statistical significance of differences was evaluated using a paired two-tailed Student's t-test, and significance was accepted when P<0.05.

### Chemicals

Histamine dihydrochloride, ovalbumin (grade III), aluminium hydroxide, L-arginine hydrochloride and D-arginine hydrochloride were obtained from Sigma Chemical Co. Saline was purchased from Braun (The Netherlands). 2 (S) -amino-6-boronohexanoic acid was provided by Organon (Oss, The Netherlands).

### RESULTS

### Reversion protocol

Fig. 3 shows that ovalbumin induces a significant AHR after both the EAR and LAR, as indicated by significantly decreased PC₁₀₀ values for histamine after these reactions. Inhalation of saline did not affect basal reactivity to histamine nor allergen-induced AHR after the EAR and LAR.

Inhalation of the arginase inhibitor ABH was without effect on basal airway responsiveness, but reversed the allergen-induced AHR after the EAR, as indicated by the significantly increased PC₁₀₀ value compared to the control measurement after this reaction.

In addition, a trend towards a reduction in the AHR after the LAR was observed, while no significant AHR was present anymore after the ABH inhalation when compared to basal responsiveness (Fig. 3).

Fig. 4 demonstrates that ABH reduces the allergen-induced AHR, expressed as PC₁₀₀ ratio pre/post challenge, from 4.77 ± 0.56-fold to 2.04 ± 0.34-fold (P<0.001) after the EAR and from 1.95 ± 0.23-fold to 1.56 ± 0.47-fold (P<0.10) after the LAR.

As ABH, inhalation of L-arginine did not affect basal airway reactivity to histamine (Fig. 5). Remarkably, the AHR after the EAR and after the LAR were reversed to a similar extent as with ABH (Figs. 5 and 6).

As with saline, inhalation of the biologically inactive D-enantiomer of arginine did not affect basal airway responsiveness and allergen-induced AHR at all (Fig. 7).

### Protection protocol

Interestingly, pretreatment with 25 mM ABH 0.5 h before allergen-challenge caused significant protection against the AHR after the EAR as compared to saline control treatment (Fig. 8). Moreover, an additional inhalation of 25 mM ABH at 8 h after allergen challenge almost completely prevented the occurrence of AHR after the LAR. Pretreatment with saline did not affect the AHR after the EAR or LAR (Fig. 8).

Fig. 9 shows that ABH significantly reduced the allergen-induced AHR after the EAR from 6.33 ± 1.30-fold (saline control, week 1) to 3.05 ± 0.51-fold (P<0.05) and from 2.08 ± 0.31-fold to 1.41 ± 0.25-fold (P<0.005) after the LAR.

Remarkably, after pretreatment with ABH in week 2 a 32.8-fold higher concentration of ovalbumin (1.31 ± 0.69 mg) was needed to induce airways obstruction compared to saline treatment of the same animals in week 1 (0.04 ± 0.01 mg; P<0.01), indicating that ABH considerably diminishes the sensitivity to the allergen. No significant increase in ovalbumin dose was observed for saline-treated animals (Fig. 10).

For the full appreciation of the ABH effect at a normally obstructive allergen load, provocations were performed with equal doses of allergen in the saline-treated (challenged to obstruction) and ABH-treated animals. Under this condition, pretreatment with ABH at 0.5 h before
allergen challenge caused a more pronounced protection against the allergen-induced AHR after the EAR, while an additional treatment with ABH at 8 h after allergen challenge completely normalized the airway responsiveness after the LAR to the basal level (Fig. 11, Table 1). Thus, treatment with ABH reduced the allergen-induced AHR after the EAR from 4.14 ± 0.59-fold (saline-treated) to 1.58 ± 0.24-fold; P<0.01) and completely prevented the development of AHR after the LAR (from 1.68 ± 0.14-fold in saline-treated animals to 1.02 ± 0.02-fold in ABH treated animals; P<0.005).

Representative on-line recordings of Pₚₗ in allergen-challenged guinea pigs are shown in Fig. 12. Compared to saline-treatment, ABH treatment at the same allergen dose greatly reduced the EAR as well as the LAR. As expected, a highly significant reduction of the initial peak response in Pₚₗ, reflecting the allergen-induced acute bronchial obstruction, was observed (P<0.01, Fig. 12), while similarly the AUCs of both the EAR and LAR were significantly reduced (Table 1).

**Table 1**

| Protective effects of ABH (25 inM nebulizer concentration) on the allergen-induced early (EAR) and late (LAR) asthmatic reactions and airway hyperresponsiveness (AHR) after these reactions. | | | | |
|---|---|---|---|---|
| Treatment^{I} | AUC^{II}(% x 5 min) | | AHR₁₀₀ (PC₁₀₀ ratio pre/post allergen challenge) | |
| | EAR | LAR | after EAR | after LAR |
| Saline | 3687±907 | 8156±2409 | 4.14±0.59 | 1.68±:0.14 |
| ABH | 983±345* | 1697±1043* | 1.58±0.24** | 1.02±0.02*** |

^{I}Saline (control) or ABH were inhaled 0.5 h before and 8 h after allergen challenge, using an equal allergen dose for both treatment groups. ^{II}The magnitudes of the EAR and the LAR are presented as the AUCs under the % change in Pp1 time-response curve between 0 and 5 h and between 8 and 24 h after allergen exposure, respectively. ^{III}Histamine PC₁₀₀-values were determined 24 h before and 6 h (after EAR) and 24 h (after LAR) following allergen challenge. Data represent means ± SEM of 5-6 animals. *P<0.05, **P<0.01 and ***P<0.005 versus saline-treated animals.

### CONCLUSION

Using the guinea pig model of allergic asthma, it was demonstrated herein that inhalation of the specific, isoenzyme-nonselective arginase inhibitor ABH acutely reversed the allergen-induced AHR after the EAR and LAR, while pretreatment with the arginase inhibitor considerably reduced the sensitivity of the airways to inhaled allergen and protected against the development of allergen-induced EAR and LAR, and AHR after both reactions.

Without being bound to theory it is assumed that inhalation of ABH acutely reverses allergen-induced AHR after the EAR and LAR by attenuating arginase-induced substrate deficiency to NO synthase isozymes in the airways. In addition, by increasing substrate availability to NO
synthases and subsequently increasing NO production, ABH may attenuate allergen-induced (particularly mast cell-derived) mediator release into airways. Several studies in asthmatic patients have indicated that a deficiency of bronchodilating (cNOS-derived) NO is involved in the development of allergen-induced AHR. Moreover, increased arginase expression or activity have been observed in airways and blood of asthmatic patients, respectively. The above results are thus indicative for the utility of the prophylactic treatment with ABH in asthmatic patients.

## Claims

1. Arginase inhibitor for use in the prophylactic maintenance therapy of an asthmatic
or allergic patient by reducing the sensitivity of the airways to inhaled allergen and protecting against the development of allergen-induced early asthmatic reaction (EAR) and late asthmatic reaction (LAR), wherein the arginase inhibitor is administered by topical inhalation.

2. Arginase inhibitor for use as claimed in claim 1, wherein the arginase inhibitor is
selected from the group consisting of 2(S)-amino-6-boronohexanoic acid (ABH), S-(2-boronoethyl)-L-cysteine (BEC), Nω-OH-L-arginine (NOHA), Nω-hydroxy-nor-L-arginine (nor-NOHA), α-difluoromethylornithine (DFMO),L-norvaline, iodoacetyl-L-ornithine, iodoacetyl-L-lysine, L-lysine, L-citrulline.

3. Arginase inhibitor for use as claimed in claim 1 or 2, wherein the arginase inhibitor is the only active ingredient.

## Patentansprüche

1. Arginase-Hemmer für die Verwendung in der prophylaktischen Erhaltungstherapie eines Asthma- oder Allergie-Patienten, indem die Empfindlichkeit der Luftwege gegen ein inhaliertes Allergen verringert wird und er gegen die Entwicklung einer Allergen-induzierten frühen Asthmareaktion (EAR) und späten Asthmareaktion (LAR) geschützt wird, wobei der Arginase-Hemmer durch topische Inhalation verabreicht wird.

2. Arginase-Hemmer für die Verwendung nach Anspruch 1, wobei der Arginase-Hemmer aus der Gruppe ausgewählt wird, die besteht aus: 2(S)-Amino-6-Boronohexansäure (ABH), S-(2-Boronoethyl)-L-Cystein (BEC), Nω-OH-L-Arginin (NOHA), Nω-Hydroxy-nor-L-Arginin (nor-NOHA), α-Difluoromethylornithin (DFMO), L-Norvalin, Iodoacetyl-L-Ornithin, Iodoacetyl-L-Lysin, L-Lysin, L-Citrullin.

3. Arginase-Hemmer für die Verwendung nach Anspruch 1 oder 2, wobei der Arginase-Hemmer der einzige aktive Inhaltsstoff ist.

## Revendications

1. Inhibiteur de l'arginase pour une utilisation dans le traitement de maintien prophylactique d'un sujet asthmatique ou allergique par réduction de la sensibilité des voies respiratoires à un allergène inhalé et protection contre le développement d'une réaction asthmatique précoce (EAR) et d'une réaction asthmatique tardive (LAR) induites par l'allergène, dans lequel l'inhibiteur de l'arginase est administré par inhalation topique.

2. Inhibiteur de l'arginase pour une utilisation telle que revendiquée dans la revendication 1, dans lequel l'inhibiteur de l'arginase est choisi dans le groupe constitué de l'acide 2(S)-amino-6-borono-hexanoïque (ABH), la S-(2-boronoéthyl)-L-cystéine (BEC), la Nω-OH-L-arginine (NOHA), la Nω-hydroxy-nor-L-arginine (nor-NOHA), l' -difluorométhylornithine (DFMO), la L-norvaline, l'iodoacétyl-L-ornithine, l'iodoacétyl-L-lysine, la L-lysine, la L-citrulline.

3. Inhibiteur de l'arginase pour une utilisation telle que revendiquée dans la revendication 1 ou 2, dans lequel l'inhibiteur de l'arginase est le seul principe actif.
